# EUROPÄISCHE PATENTSCHRIFT

(11) **EP 0 440 763 B1**
(45) Veröffentlichungstag und Bekanntmachung des Hinweises auf die Patenterteilung: **22.03.1995**
(21) Anmeldenummer: 90912217.8
(22) Anmeldetag: 22.08.1990
(51) Int. Cl.: C12N 15/58, C12N 9/64, C12N 5/10, A61K 38/48

(54) **t-PA-MUTANTE GK1L**
t-PA MUTANT GK1L
GK1L MUTANTE D'ACTIVATEUR TISSULAIRE DU PLASMINOGENE

(30) Priorität: 23.08.1989 DE 3927865
(43) Veröffentlichungstag der Anmeldung: 14.08.1991
(73) Patentinhaber: BOEHRINGER MANNHEIM GMBH, 68298 Mannheim (DE)
(72) Erfinder: WEIDLE, Ulrich, D-8000 München 2 (DE); STERN, Anne, D-8122 Penzberg (DE)
(74) Vertreter: Huber, Bernhard, Dipl.-Chem.
(86) Internationale Anmeldenummer: EP9001401
(87) Internationale Veröffentlichungsnummer: WO9102798

(56) Entgegenhaltungen:
- WO-A-87/03906
- Gene, Band 79, Nr. 2, 1989, Elsevier Science Publishers B.V.(Biomedical Division), (Amsterdam, NL), A. Stern et al., pp. 333-344
- Febs Letters, Band 189, Nr. 1, September 1985, published by Elsevier Science Publishers B.V. (Biomedical Division), Federation of European Biochemical Societies, (Amsterdam, NL), H. Kagitani et al., pp. 145-149
- Gene, Band 87, Nr. 2, 15. März 1990, Elsevier Science Publishers B.V. (Biomedical Division), (Amsterdam, NL), A. Stern et al., pp. 305-308

## Beschreibung

Human Tissue Plasminogen Activator (t-PA) ist eine Serinprotease mit einem Molekulargewicht von 68.000 Dalton, die das Proenzym Plasminogen in die aktive Serinprotease Plasmin umwandelt. Plasmin löst Fibrin auf, das die Hauptkomponente der Proteinmatrix vongeronnenem Blut ist. t-PA besitzt eine hohe Affinität für Fibrin und wird auch durch Fibrin aktiviert (siehe Fibrinolysis 2 (1988), 133-142). An t-PA besteht daher ein großes medizinisches Interesse.

Ein Vorteil von t-PA gegenüber anderen bekannten Plasminogen-Aktivatoren, wie z.B. Urokinase oder Streptokinase, ist die Stimulierbarkeit seiner katalytischen Aktivität durch Fibrin (siehe J. Biol. Chem. 257 (1982), 2912-2919; Biochem. Biophys. Acta 755 (1983) 531-533).

t-PA (Aminosäuresequenz vgl. Vehar et al., Bio/Technology 2 (1984) 1051-1057) besteht in seiner einkettigen Form aus einer schweren Kette (H-Kette) und einer leichten Kette (L-Kette), die durch eine Disulfidbrücke zusammengehalten werden. Die zweikettige Form entsteht aus einer einkettigen Vorläuferform durch spezifische Spaltung mit Plasmin oder anderen Proteasen zwischen den Aminosäuren (A.S.) 275 und 276. Die 32.000 Dalton schwere L-Kette enthält den enzymatisch aktiven Bereich, der Homologien zu anderen Serinproteasen wie Urokinase oder Plasmin besitzt (Proc. Natl. Acad. Sci. USA 81 (1984), 5335-5339). Die Domänen auf der 39.000 Dalton schweren H-Kette sind die Fingerdomäne (F) mit Homologie zu Fibronektin (A.S. 1-49), die Wachstumsfaktordomäne (G) mit Homologie zu Maus- und menschlichem epidermalem Wachstumsfaktor (A.S. 50-87) und zwei Kringeldomänen K1 (A.S. 88-175) und K2 (A.S. 176-262) mit Homologie zu den Kringelstrukturen in Plasminogen.

Zur Funktion der einzelnen Domänen der H-Kette ist bereits bekannt, daß nur die Domänen K2 oder/und F, aber nicht die Domäne K1 für die Bindung von t-PA an Fibrin und somit auch für die Stimulierung der katalytischen Aktivität von t-PA in Gegenwart von Fibrin verantwortlich sind (EP-A-0 234 051).

Aus EMBO 7 (1988) 2731-2740 ist bekannt, daß auch die Aktivität einer t-PA-Mutante, die aus der H-Kette die vollständigen Domänen K1 und F, sowie einen Teil der Domäne K2 enthält, durch Fibrin stimulierbar ist.

Somit bleibt es offen, in welchem Ausmaß einzelne Domänen der H-Kette die Aktivität des t-PA-Moleküls für Fibrin bewirken und eine Stimulierung der Plasminogenspaltenden Aktivität hervorrufen.

Aufgabe der Erfindung ist es, eine t-PA-Mutante zur Verfügung zu stellen, die im Vergleich zu t-PA eine größere Plasminogen-spaltende Aktivität besitzt und deren katalytische Aktivität ebenfalls durch Fibrin oder Fibrinogen stimulierbar ist.

Die Aufgabe wird erfindungsgemäß durch Herstellung einer rekombinanten DNA gelöst, die für ein Protein mit den Domänen GK1L von t-PA kodiert, wobei die für die Domänen K2 und F kodierenden Sequenzen oder die im Rahmen der Degeneration des genetischen Codes davon abgeleiteten Sequenzen, d.h. entsprechend der genauen Exon/Intron-Grenzen auf dem t-PA-Gen vollständig deletiert sind. In der erfindungsgemäßen rekombinanten DNA fehlen somit die Nukleotide 715 bis 972 und 199 bis 339 der t-PA cDNA (Numerierung nach Nature 301, (1983), 214-221). Obwohl die für die Stimulierbarkeit der Aktivität als erheblich angesehenen Domänen K2 und F der H-Kette bei der t-PA-Mutante GK1L dem Genprodukt der erfindungsgemäßen rekombinanten DNA vollständig fehlen, bleibt bei GK1L die Stimulierbarkeit der katalytischen Aktivität durch Fibrin überraschenderweise erhalten. Überraschenderweise ist sogar die katalytische Aktivität eines Überstands aus Zellen, die GK1L exprimieren, deutlich höher als die Aktivität eines Überstands aus Zellen, die t-PA exprimieren.

Ein Gegenstand der Erfindung ist auch ein Verfahren zur Herstellung von erfindungsgemäßer rekombinanter DNA aus einer für t-PA oder für eine t-PA-Mutante, welche mehr als die Domänen G, K1 und L enthält, kodierenden DNA-Sequenz zur Deletion derjenigen Sequenzen, welche nicht für die Domänen G, K1 und L kodieren, unter Einhaltung der genauen Exon/Intron-Grenzen auf dem t-PA-Gen. Besonders bevorzugt ist ein Verfahren, bei dem die Deletion der für die Domänen K2 und F kodierenden DNA durch gerichtete Mutagenese geschieht.

Ein Gegenstand der Erfindung ist auch ein rekombinanter Vektor, der eine oder mehrere Kopien der erfindungsgemäßen rekombinanten DNA enthält. Eine bevorzugte Ausführungsform ist dabei ein Vektor, der für die Expression der rekombinanten DNA in eukaryontischen Zellen geeignet ist. Eine besonders bevorzugte Ausführungsform der Erfindung ist ein eukaryontischer Vektor mit dem GK1L-Gen, der den frühen SV40-Promotor und ein Maus-dhfr-Gen enthält. Am meisten bevorzugt ist jedoch das erfindungsgemäße Plasmid pSV-GK1L.

Ein Gegenstand der Erfindung ist weiterhin eine Zellinie, die mit der erfindungsgemäßen rekombinanten DNA oder einem erfindungsgemäßen Vektor transformiert ist. Besonders bevorzugt ist eine eukaryontische Zellinie, am meisten bevorzugt ist eine CHO-dhfr⁻-Zellinie (z.B. ECACC 88072103), die eine erfindungsgemäße rekombinante DNA bzw. einen erfindungsgemäßen Vektor enthält.

Ein Gegenstand der Erfindung ist auch ein Protein mit fibrinolytischen Eigenschaften, das aus den Aminosäuresequenzen der Domänen G, K1 und L von t-PA in dieser Reihenfolge besteht und gegebenenfalls glykosiliert ist. Die Erfindung beinhaltet ebenfalls ein Verfahren zur Herstellung eines Proteins mit fibrinolytischen Eigenschaften, wobei man eine erfindungsgemäße rekombinante DNA oder einen erfindungsgemäßen rekombinanten Vektor in geeigneten Wirtszellen exprimiert und das Expressionsprodukt aus dem Kulturmedium oder durch Aufschluß der Wirtszellen gewinnt. Bevorzugt ist dabei ein Verfahren, bei dem das erfindungsgemäße Protein aus eukaryontischen Wirtszellen, vorzugsweise CHO dhfr⁻-Wirtszellen, in glykosilierter Form gewonnen wird. Überraschenderweise besitzt ein Überstand aus CHO dhfr⁻-Zellen, die mit dem erfindungsgemäßen Plasmid pSV-GK1L transformiert sind und GK1L sekretieren, eine höhere katalytische Aktivität als ein Überstand aus Zellen, die mit einem entsprechenden Expressionsvektor pSV-FGK1K2L transformiert sind, auf dem sich das Wildtyp t-PA-Gen befindet.

Besonders bevorzugt ist ein Verfahren, bei dem Wirtszellen verwendet werden, die in einem Aprotinin enthaltenden Medium kultiviert werden.

Dabei sind überraschenderweise sowohl die Aktivität und das Ausmaß der Stimulierung durch Fibrin im Überstand von Wirtszellen höher, deren Kulturmedium Aprotinin enthält.

Gegenstand der Erfindung ist schließlich ein fibrinolytisches Mittel, das ein erfindungsgemäßes Protein enthält.

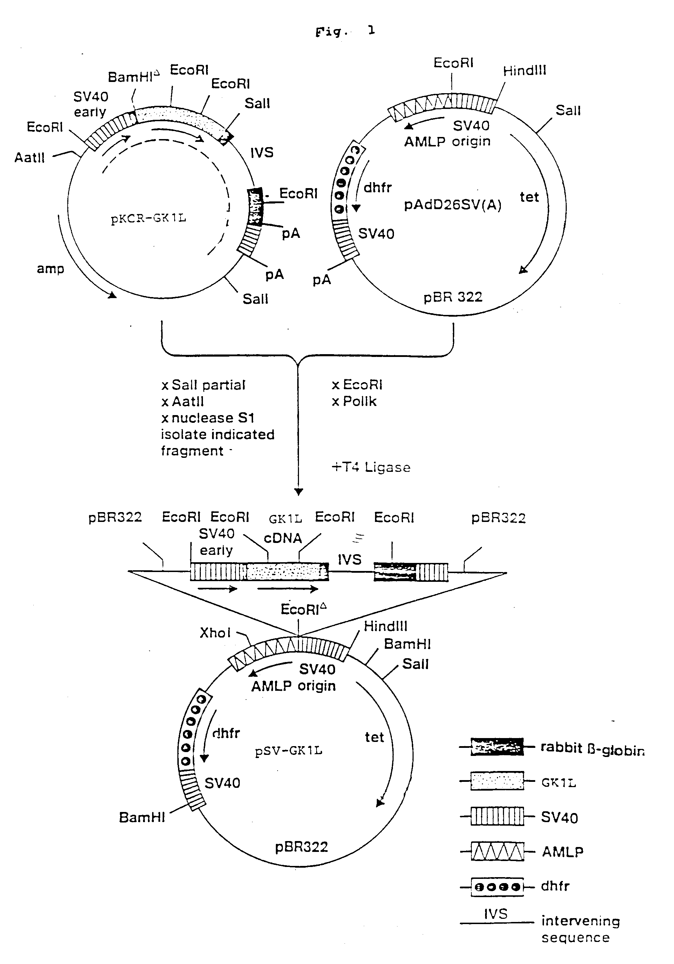

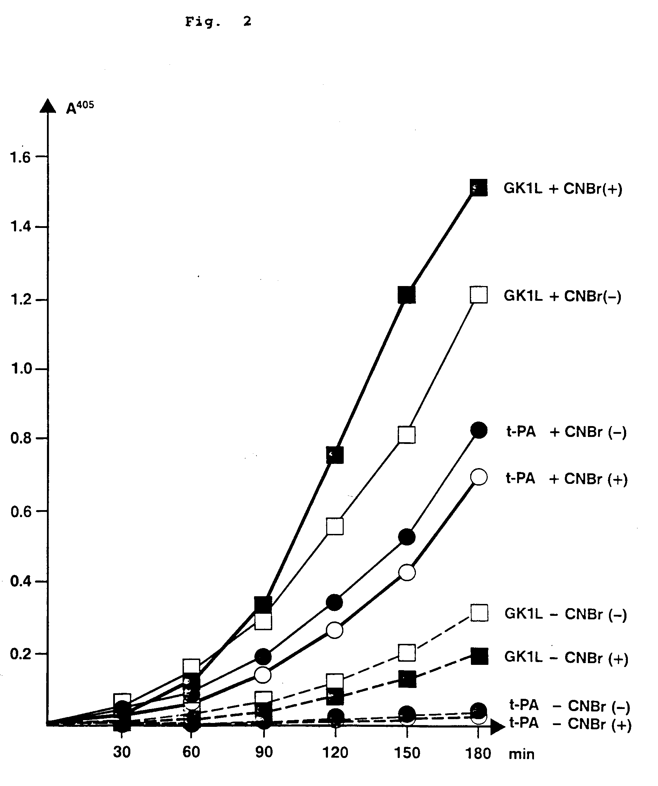

Folgende Beispiele sollen die Erfindung in Verbindung mit den Figuren 1 und 2 näher erläutern.

Es zeigen:
- Fig. 1.: die Herstellung des Plasmids pSV-GK1L,
- Fig. 2: den Vergleich der Fibrinogen-stimulierten katalytischen Aktivität von Wildtyp-t-PA und GK1L.

### Beispiel 1

### Herstellung eines t-PA-Derivats, bei dem die Finger- und Kringel 2-Domäne deletiert sind (GK1L)

Die Herstellung der Deletionsmutante FGK1L aus t-PA cDNA geschah durch Deletion der Kringel 2-Domäne mit gerichteter Mutagenese nach dem Verfahren aus Bio/Technology 2 (1984), 636-639. Als Ausgangsplasmid diente pePA 133 (Herstellung siehe EP-A 0242836), auf dem sich die t-PA Nukleotidsequenz 190-1809 befindet. Der Mutagenese-Primer 1 (5'GCCTGCTCTGAGTCCACCTGCGGC3') wurde verwendet, um die Nukleotide 715 bis 972 (Exons VIII und IX) zu entfernen (entsprechend der Numerierung der t-PA-cDNA in Nature 301 (1983), 214-221). Ein Plasmid p7745, das für die Deletionsmutante FGK1L kodiert, wurde dann durch Koloniehybridisierung mit dem Mutageneseprimer 1 isoliert und sequenziert. Zur späteren Expression in eukaryontischen Zellen war eine Rekonstitution der t-PA Signalsequenz erforderlich.

Zunächst wurde die FGK1L cDNA mit der Leader-Sequenz und der 3'UT (3' untranslatierte Region), entsprechend der originalen cDNA-Sequenz, versehen. Dazu wurde Plasmid p7.1, DSM 4719, das im Polylinker von pUC12 die 5'UT (5' untranslatierte Region) bis zu Position 77, die Leadersequenz und die N-terminale Sequenz von t-PA bis einschließlich Nukleotidposition 208 enthielt, mit PstI und Hind III gespalten (ca. 2,7 kb). Zusätzlich wurden folgende t-PA cDNA Sequenzen enthaltenden Fragmente isoliert: aus p7745 ein Pst I/Hae II-Fragment, das die Nukleotidpositionen 209 bis 421 umfaßt sowie ein Hae II/EcoRI-Fragment mit den Nukleotidpositionen 421 bis 1273, wobei durch die Mutagenese die Nukleotidpositionen 715 bis 972 deletiert sind und aus pePA 98,1 (Herstellung siehe EP-A 0 242 836), ein Eco RI/Hind III-Fragment mit den Nukleotidpositionen 1274-2165.

Die Fragmente wurden ligiert und in E.coli, DSM 3689, transformiert. Plasmid-tragende Transformanten wurden durch Zugabe von 50 »g/ml Ampicillin im Nährmedium selektioniert. Das richtige Plasmid, mit pePA 159 bezeichnet, wurde durch Restriktionsenzym-Analyse verifiziert. Aus diesem Plasmid konnte die FGK1L c-DNA als XbaI-HindIII-Fragment (mit Signal-Sequenz, aber ohne eigene Polyadenylierungs-Stelle) isoliert werden. Dieses Fragment enthält sieben Nukleotide 5' untranslatierte Region (5'UT) und die 3' untranslatierte Region (3'UT) bis zur BglII-Stelle bei Position 2160 (Nature 301 (1983) 214-221).

Zur Deletion der Fingerdomäne F (Exon IV) aus FGK1L wurde der Mutagenese-Primer 2 (5'GATCTTACCAATGCAGCGAGC3') verwendet. Durch gerichtete Mutagenese wurden die Nukleotide 199 bis 339 auf der t-PA-cDNA entfernt. Ein Plasmid mit der rekombinanten DNA mit der Domänenzusammensetzung GK1L wurde durch Koloniehybridisierung mit Mutagenese-Primer 2 isoliert und sequenziert. Dabei wurde wie folgt verfahren: Die Filter mit immobilisierter DNA wurden 4 Stunden lang bei 65°C in 0,2 % SDS, 1,0 % Sarkosyl^{R}, 4x SET (0,6 mmol/l NaCl, 0,2 mol/l Tris-HCl, pH 8,0, 4 mmol/l EDTA) und 4x Denhardts-Lösung (0,08 % Ficoll^{R}, 0,08 % Polyvinylpyrrolidon, 0,08 % Rinderserumalbumin) vorhybridisiert.
Die Hybridisierung erfolgte 12 Stunden bei 46°C in 0,2 % SDS, 1,0 % Sarkosyl^{R}, 4x SET, 4x Denhardts und mit 5·10⁶ cpm kinasiertem Mutageneseprimer pro Filter. Die Filter wurden 3 x 5 Minuten bei Raumtemperatur, anschließend 1 x 10 Minuten bei 37°C und 1 x 5 Minuten bei 50°C in 4x SET, 0,2 % SDS gewaschen.

### Beispiel 2

### Immunologische Charakterisierung von GK1L aus CHO-Zellen

t-PA-cDNA und GK1L-cDNA wurden in die einzige BamHI-Schnittstelle des Plasmids pKCR (Proc.Natl.Acad.Sci. USA 78 (1981), 1527-1531) als XbaI-HindIII-Fragmente insertiert, woraus sich die Plasmimde pKCR-FGK1K2L und pKCR-GK1L ergaben. Die Enden der Fragmente wurden hierfür mit Polymerase I Klenow-Fragment aufgefüllt. Beide c-DNAs enthielten sieben authentische Nukleotide am 5'-Ende, und es fehlten ihre eigenen Polyadenylierungsstellen. Beide Plasmide verliehen Bakterien Resistenz gegen das Antibiotikum Ampicillin (Amp). Die Expression beider c-DNAs wird durch den SV40 early-Promotor getrieben. Auf die c-DNA folgt im Plasmid das große Intron von Kaninchen-β-Globin und Polyadenilierungsstellen von Kaninchen-β-Globin und SV40. Um diese Expressionskassette zu isolieren, wurden pKCR-FGK1K2L und pKCR-GK1L durch partielle Spaltung mit SalI linearisiert und dann mit AatII geschnitten und die überhängenden Enden mit Nuklease S1 abgebaut. Das genannte Fragment wurde aus einem niedrigschmelzenden Agarosegel isoliert und in die aufgefüllte einzige EcoRI-Schnittstelle von pAdD26SV(A) (J.Mol.Biol. 159 (1982) 601-621) einligiert. pAdD26SV(A) enthält eine Expressionskassette für Maus-DHFR-c-DNA, die durch den major late-promotor des Adenovirus 2 (AMLP) getrieben wird, den SV40-Replikationsursprung und verleiht Bakterien Resistenz gegen das Antibiotikum Tetracyclin. Die Orientierung der Expressionskassette für t-PA in den resultierenden Plasmiden pSV-FGK1K2L und pSV-GK1L, wurde durch Restriktionsanalyse überprüft. Fig. 1 zeigt schematisch die Herstellung des Plasmids pSV-GK1L sowie die Lage der einzelnen Elemente auf dem Plasmid.

Mit den rekombinanten Vektoren pSV-FGK1K2L und pSV-GK1L wurden CHO dhfr⁻-Zellen (ECACC 88072103) transformiert (Proc. Natl. Acad. Sci. USA 76 (1979), 4350-4354). Dazu wurden Calciumphosphat-Präzipitate mit 20 »g pSV-FGK1K2L bzw. pSV-GK1L in einem Volumen von 4 ml hergestellt (Virology 52 (1973), 456-467). 1 ml des Präzipitats wurde zu 3 x 10⁵ bis 1 x 10⁶ Zellen in 10 ml Medium zugegeben. Die Zellen wurden 8 - 16 Stunden inkubiert, dann das Medium entfernt, die Zellen mit 10 ml TBS (25 mmol/l Tris-HCl, pH 7,4, 137 mmol/l NaCl, 5 mmol/l KCl, 0,6 mmol/l NaH₂PO₄ gewaschen und dann in geeignetem Medium inkubiert. CHO-dhfr⁻-Zellen (ECACC 88072103) wurden 48 Stunden nach Transfektion 1:10 umgesetzt und dann in einem Selektionsmedium gezüchtet (J.Mol.Biol. 159 (1982) 601-621). Die entstandenen Klone wurden 2 bis 3 Wochen nach der Transfektion unter Zuhilfenahme eines Klonierungszylinders trypsinisiert, zur Massenkultur hochgezogen und die Überstände auf t-PA-Immunreaktivität durch ELISA untersucht (Gene 51 (1987) 31-41). Positive Klone wurden in einem Medium mit 20 nmol/l Methotrexat inkubiert. Nach 2 Wochen erschienen Methotrexat-resistente Kolonien. Sie wurden zur Konfluenz gezüchtet und 100 nmol/l Methotrexat im Medium ausgesetzt. Resistente Zellen wurden einer schrittweise höher werdenden Methotrexat-Konzentration (300 nmol/l, 500 nmol/l, 1 »mol/l und 5 »mol/l) ausgesetzt. Klone wurden durch limitierende Verdünnung isoliert und die besten t-PA Produzenten ausgewählt.

CHO-Zellen, die eine konstitutive Sekretion von Wildtypt-PA oder GK1L zeigten, wurden in DMEM-Medium (Dulbecco's modified Eagle Medium), supplementiert mit 10 % foetalem Kalbserum, in Anwesenheit und Abwesenheit von Aprotinin (50 »g/ml) gezüchtet. Die Überstände wurden auf 0,3 mol/l Arginin, pH 7,5, mit HCl eingestellt und auf eine ETI-Sepharosesäule aufgebracht (J. Biol. Chem. 259 (1984) 11635-11638). Die Proteine wurden mit 20 mmol/l Citratpuffer, pH 2,5, eluiert und anschließend gegen 20 mmol/l Tris-HCl, pH 7,5, dialysiert.

Aliquots der gereinigten Proteine wurden unter Zusatz von 10 »g Cytochrom c eine Stunde lang mit 4 Volumina Aceton bei -20°C präzipitiert und anschließend in Laemmli-Probenpuffer gelöst. Die Proteinproben wurden 3 Minuten lang gekocht und auf einem 12,5%igen SDS-Polyacrylamidgel unter Verwendung eines diskontinuierlichen Puffersystems aufgetrennt (Laemmli, Nature 227 (1970), 680-685). Nach der Elektrophorese wurden die Gele auf Nitrocellulosefilter elektrogeblottet. Die Filter wurden mit TBS gewaschen und dann bei Raumtemperatur 30 bis 60 Minuten lang mit TBS + 0,05 % Tween + 3 % Gelatine abgesättigt und schließlich kurz mit Wasser gewaschen. Danach wurden die Membranfilter mit einer 1:1000 Verdünnung eines Peroxidase-konjugierten Ziegen-Antikörpers gegen menschliches t-PA eine Stunde lang bei Raumtemperatur in TBS + 0,5 % Rinderserumalbumin behandelt. Um die Immunkomplexe sichtbar zu machen, wurden die Filter nach drei weiteren Waschgängen mit TBS mit einer 1:1 Lösung aus 2,5 mmol/l Tetramethylbenzidin und 4,5 mmol/l Natriumdioctylsulfosuccinat in Methanol, und 0,005 % Wasserstoffperoxid in 0,1 mol/l Zitronensäurepuffer, pH 5, inkubiert. Als Marker für die Polyacrylamid-Gelelektrophorese wurde der Rainbow-Mix (Amersham) verwendet, der folgende Proteine enthält: Myosin, 200 kD, Phosphorylase (b), 92,5 kD, Rinderserumalbumin, 69 kD, Ovalbumin, 46 kD, Carboanhydrase, 30 kD, Trypsininhibitor, 21,5 kD und Lysozym, 14 kD.

Der Kulturüberstand von Zellen, die das GK1L exprimierende Plasmid pSV-GK1L enthalten, ergibt bei Behandlung mit Antikörpern gegen t-PA eine immunreaktive Bande von ungefähr 50.000 Dalton (entsprechend der einkettigen Form von GK1L), eine Bande von ungefähr 31.000 Dalton (entsprechend der L-Kette) und eine Bande von ungefähr 19.000 Dalton (entsprechend der H-Kette). Der Anteil an zweikettigen Molekülen nimmt gegenüber den einkettigen Molekülen ab, wenn das Zellmedium den Proteasehemmstoff Aprotinin enthält.

Dagegen weist t-PA eine Bande von 65.000 bis 68.000 Dalton (entsprechend der einkettigen Form) und 2 Banden von 34.000 bzw. 31.000 Dalton (entsprechend der H- bzw. L-Kette) auf.

### Beispiel 3

### Vergleich der Fibrinogen-stimulierten katalytischen Aktivitäten von t-PA und GK1L

t-PA und GK1L wurden aus den Überständen von CHO-Zellen angereichert, wie in Beispiel 2 beschrieben. Die CHO-Zellen, die t-PA oder GK1L sekretieren, wurden dabei in Gegenwart oder Abwesenheit von Aprotinin (50 »g/ml) gezüchtet. Zur Durchführung des Aktivitäts-Tests wurden die Überstände 1:250 verdünnt, was eine vernachlässigbar geringe Inhibitorkonzentration zur Folge hatte. Die Plasminogen-spaltende Aktivität wurde durch einen indirekten spektrophotometrischen Test bestimmt (Thromb. Haemostasis 48 (1982), 266-269). t-PA wandelt Plasminogen in die aktive Serinprotease Plasmin um, die eine Bindung eines chromogenen Substrats hydrolysiert, dessen Absorption bei 405 nm für eine Zeitdauer von bis zu 3 Stunden gemessen wurde. In modifizierten Experimenten wurde tosyliertes Gly-Pro-Lys-p-Nitroanilid (Chromozym® PL) als chromogenes Substrat verwendet. Die Tests wurden bei 25°C in 0,1 mol/l Tris-HCl, pH 7,5, 0,15 mol/l Tween 80 und 0,13 »mol/l Plasminogen und 0,30 mmol/l Chromozym®PL durchgeführt, in Abwesenheit oder Gegenwart von mit CNBr gespaltenem Fibrinogen (120 »g/ml). Als Maß für die Freisetzung von p-Nitrophenol aus dem chromogenen Substrat wurde die Extinktion bei 405 nm bestimmt und als Funktion der Inkubationsdauer aufgezeichnet. Die Ergebnisse zeigt Fig. 2.

Die Plasminogen-spaltende Aktivität von t-PA (isoliert aus aprotininfreien Überständen) ist mit gefüllten Kreisen dargestellt, die Aktivität von t-PA (isoliert aus Aprotinin enthaltenden Überständen) ist mit offenen Kreisen dargestellt, die Aktivität von GK1L (isoliert aus Aprotinin enthaltenden Überständen) ist in gefüllten Quadraten dargestellt und die Aktivität von GK1L (isoliert aus Überständen ohne Aprotinin) ist durch offene Quadrate dargestellt. ± CNBr zeigt die Gegenwart oder Abwesenheit von CNBr-behandelten Fibrinogen-Fragmenten im Test. (+) oder (-) zeigt an, ob das Protein aus Aprotinin enthaltenden oder Aprotinin-freien Gewebekulturüberständen gereinigt wurde.

Aus Fig. 2 ist ersichtlich, daß in Gegenwart von Fibrinogen eine Stimulierung der katalytischen Aktivität von GK1L erfolgt.

Dabei ist überraschenderweise die katalytische Aktivität eines Überstands aus Zellen, die GK1L exprimieren, mit und ohne Fibrinogen deutlich höher als die eines Überstandes aus Zellen, die Wildtyp t-PA exprimieren. Bei Anwesenheit von Aprotinin im Medium ist die Aktivität von GK1L in Gegenwart von Fibrinogen höher und ohne Fibrinogen niedriger als ohne Aprotinin.

## Patentansprüche

1. Rekombinante DNA, **dadurch gekennzeichnet,** daß sie für ein Protein mit den Domänen GK1L von t-PA kodiert, wobei die für die Domänen K2 und F des Wildtyp t-PA-Gens kodierenden Sequenzen oder die im Rahmen der Degeneration des genetischen Codes davon abgeleiteten Sequenzen entsprechend der genauen Exon/Intron-Grenzen auf dem t-PA-Gen, vollständig deletiert sind.

2. Verfahren zur Herstellung rekombinanter DNA nach Anspruch 1, **dadurch gekennzeichnet,** daß man aus einer für Wildtyp t-PA oder eine t-PA-Mutante, welche mehr als die Domänen G, K1 und L enthält, kodierenden DNA-Sequenz unter Einhaltung der genauen Exon/Intron-Grenzen auf dem t-PA-Gen diejenigen Sequenzen deletiert, welche nicht für die Domänen G, K1 und L kodieren.

3. Verfahren nach Anspruch 2, **dadurch gekennzeichnet,** daß man die Deletion der für die Domänen F und K2 kodierenden Sequenzen durch gerichtete Mutagenese durchführt.

4. Rekombinanter Vektor, **dadurch gekennzeichnet,** daß er integriert eine oder mehrere Kopien der rekombinanten DNA nach Anspruch 1 enthält.

5. Rekombinanter Vektor nach Anspruch 4, **dadurch gekennzeichnet,** daß er den frühen SV40 Promotor und ein Maus dhfr-Gen enthält.

6. Plasmid pSV-GK1L gemäß Fig. 1.

7. Zellinie, **dadurch gekennzeichnet,** daß sie mit einer rekombinanten DNA nach Anspruch 1 oder einem Vektor nach einem der Ansprüche 4 bis 6 transformiert ist.

8. Zellinie nach Anspruch 7, **dadurch gekennzeichnet,** daß sie eukaryontisch, vorzugsweise eine CHO dhfr⁻-Zellinie, ist.

9. Protein mit fibrinolytischen Eigenschaften, **dadurch gekennzeichnet,** daß es aus den Aminosäuresequenzen der Domänen G, K1 und L von t-PA in dieser Reihenfolge besteht und gegebenenfalls glykosiliert ist.

10. Verfahren zur Herstellung eines Proteins mit fibrinolytischen Eigenschaften, **dadurch gekennzeichnet,** daß man eine DNA nach Anspruch 1 oder einen Vektor nach einem der Ansprüche 4 bis 6 in geeigneten Wirtszellen exprimiert und das Expressionsprodukt aus dem Kulturmedium oder durch Aufschluß der Wirtszellen gewinnt.

11. Verfahren nach Anspruch 10, **dadurch gekennzeichnet,** daß man eukaryontische, vorzugsweise CHO-dhfr⁻-Zellen (ECACC 88072103) als Wirtszellen verwendet und ein glykosiliertes Produkt gewinnt.

12. Verfahren nach Anspruch 10 oder 11, **dadurch gekennzeichnet,** daß man Wirtszellen verwendet, die in einem Aprotinin-enthaltenden Medium kultiviert werden.

13. Fibrinolytisches Mittel, enthaltend ein Protein nach Anspruch 9.

## Claims

1. Recombinant DNA,
**wherein**
it codes for a protein with the domains GK1L of t-PA in which the sequences coding for the domains K2 and F of the wild-type t-PA gene or sequences derived therefrom within the scope of the degeneration of the genetic code are completely deleted in accordance with the exact exon/intron borders on the t-PA gene.

2. Process for the production of recombinant DNA as claimed in claim 1,
**wherein**
those sequences which do not code for the domains G, K1 and L are deleted from a DNA sequence coding for wild-type t-PA or for a t-PA mutant which contains more than the domains G, K1 and L, while maintaining the exact exon/intron borders on the t-PA gene.

3. Process as claimed in claim 2,
**wherein**
the deletion of the sequences coding for the domains F and K2 is carried out by site-directed mutagenesis.

4. Recombinant vector,
**wherein**
it contains one or several integrated copies of the recombinant DNA as claimed in claim 1.

5. Recombinant vector as claimed in claim 4,
**wherein**
it contains the early SV40 promoter and a mouse dhfr⁻ gene.

6. Plasmid pSV-GK1L in accordance with Figure 1.

7. Cell line,
**wherein**
it is transformed with a recombinant DNA as claimed in claim 1 or with a vector as claimed in one of the claims 4 to 6.

8. Cell line as claimed in claim 1,
**wherein**
it is a eukaryotic, preferably a CHO dhfr⁻, cell line.

9. Protein with fibrinolytic properties,
**wherein**
it consists of the amino acid sequences of the G, K1 and L domains of t-PA in this order and which is glycosylated, if desired.

10. Process for the production of a protein with fibrinolytic properties,
**wherein**
a DNA as claimed in claim 1 or a vector as claimed in one of the claims 4 to 6 is expressed in suitable host cells and the expression product is obtained from the culture medium or by lysis of the host cells.

11. Process as claimed in claim 10,
**wherein**
eukaryotic, preferably CHO-dhfr⁻, cells (ECACC 88072103) are used as host cells and a glycosylated product is obtained.

12. Process as claimed in claim 10 or 11,
**wherein**
host cells are used which are cultured in a medium containing aprotinin.

13. Fibrinolytic agent containing a protein as claimed in claim 9.

## Revendications

1. ADN recombiné caractérisé en ce qu'il code une protéine présentant les domaines GK1L du t-PA, les séquences codant les domaines K2 et F du gène du t-PA de type sauvage ou les séquences qui en dérivent dans le cadre de la dégénérescence du code génétique étant totalement délétées d'une manière qui correspond aux limites exons/introns exactes sur le gène du t-PA.

2. Procédé de préparation d'un ADN recombiné selon la revendication 1, caractérisé en ce qu'à partir d'une séquence d'ADN codant un t-PA de type sauvage ou un mutant du t-PA qui contient plus que les domaines G, K1 et L, on délète les séquences qui ne codent pas les domaines G, K1 et L en respectant les limites exons/introns exactes sur le gène du t-PA.

3. Procédé selon la revendication 2, caractérisé en ce que l'on réalise la délétion des séquences codant les domaines F et K2 par mutagenèse dirigée.

4. Vecteur recombiné caractérisé en ce qu'il contient à l'état intégré une ou plusieurs copies de l'ADN recombiné selon la revendication 1.

5. Vecteur recombiné selon la revendication 4, caractérisé en ce qu'il contient le promoteur précoce de SV40 et un gène dhfr de souris.

6. Plasmide pSV-GK1L selon la figure 1.

7. Lignée cellulaire caractérisée en ce qu'elle est transformée avec un ADN recombiné selon la revendication 1 ou avec un vecteur selon l'une des revendications 4 à 6.

8. Lignée cellulaire selon la revendication 7, caractérisée en ce qu'il s'agit d'une lignée cellulaire eucaryotique, de préférence d'une lignée cellulaire CHO dhfr⁻.

9. Protéine à propriétés fibrinolytiques caractérisée en ce qu'elle consiste en les séquences d'acides aminés des domaines G, K1 et L du t-PA dans cet ordre et en ce qu'elle est éventuellement glycosylée.

10. Procédé de préparation d'une protéine à propriétés fibrinolytiques, caractérisé en ce que l'on exprime un ADN selon la revendication 1 ou un vecteur selon l'une des revendications 4 à 6 dans des cellules hôtes appropriées et on obtient le produit d'expression à partir du milieu de culture ou par lyse des cellules hôtes.

11. Procédé selon la revendication 10, caractérisé en ce que l'on utilise des cellules eucaryotiques, de préférence des cellules CHO dhfr⁻ (ECACC 88072103) comme cellules hôtes et l'on obtient un produit glycosylé.

12. Procédé selon la revendication 10 ou 11, caractérisé en ce que l'on utilise des cellules hôtes qui sont cultivées dans un milieu contenant de l'aprotinine.

13. Agent fibrinolytique contenant une protéine selon la revendication 9.
